# EUROPEAN PATENT APPLICATION

(11) **EP 1 533 366 A1**
(43) Date of publication of application: **25.05.2005**
(21) Application number: 03738515.0
(22) Date of filing: 26.06.2003
(51) Int. Cl.: C12M 1/00, C12N 11/02, C12N 5/06, A61L 27/00, D01F 8/16

(54) **CHITOSAN/ACIDIC BIOPOLYMER HYBRID FIBER AND CULTURE BASE FOR ANIMAL CELLS**

(30) Priority: 28.06.2002 JP 2002190674
(71) Applicant: Chemical Biology Institute, Sapporo-shi, Hokkaido 004-0814 (JP)
(72) Inventor: MAJIMA, Tokifumi, Sapporo-shi, Hokkaido 064-0921 (JP); IWASAKI, Norimasa, Sapporo-shi, Hokkaido 060-0003 (JP); FUNAKOSHI, Tadanao, Sapporo-shi, Hokkaido 065-0023 (JP); MINAMI, Akio, Sapporo-shi, Hokkaido 064-0959 (JP); NISHIMURA, Shin-ichiro, Sapporo-shi, Hokkaido 060-0009 (JP); TOKURA, Seiichi, Suita-shi, Osaka 564-0041 (JP); HARADA, Kazuo, Sapporo-shi, Hokkaido 004-0003 (JP); NONAKA, Sachiko, Sapporo-shi, Hokkaido 062-0907 (JP); MAEKAWA, Nobuhiko, Sapporo-shi, Hokkaido 004-0813 (JP)
(74) Representative: Goddar, Heinz J., Dr.
(86) International application number: PCT/JP2003/008080
(87) International publication number: WO 2004/003130

(57) **Abstract**

It is intended to provide a three-dimensional scaffolds appropriate for culturing animal cells such as chondrocytes and fibroblasts. These three-dimensional scaffolds which comprise a chitosan/acidic biopolymers hybrid fibers composed of chitosan or its salts in the inside of the fiber and complexes of chitosan with biodegradable acidic biopolymers coating the fiber surface, are made of fibers capable of retaining their shapes after allowing to stand Dulbecco's modified Eagle's medium (DMEM) containing 10 % of FBS (fetal bovine serum)at room temperature for 2 weeks.

## Description

### TECHNICAL FIELD

The present invention relates to chitosan/acidic biopolymer hybrid fibers and the method for preparation of the same. The present invention is also related to a three dimensional scaffolds for animal cells comprising the fibers and a method for culturing animal cells using the scaffolds.

### BACKGROUND OF THE INVENTION

We are now entering senior citizen society and the number of patients suffering from arthritis reach one percent of the population. Most of the patients have osteoarthritis or chronic rheumatism caused by damage or aneretic death of cartilage tissue. Since cartilage regeneration ability is very low, artificial prosthetic devices were implanted. However, there are many problems in this method such as inflammation by elution of metal ion from inserted material, looseness with bone joining portion and durability. Since lifetime thereof are about ten years, artificial prosthetic devices cannot be permanently curable treatment.

In the treatment of damage of ligament (in particular anterior cruciate ligament which fixes knee joint) and tendon, autologous transplantation of healthy host ligament or tendon is done. However, muscle strength on healthy host region decreases to about one half, resulting in the obstruction of exercise ability. Also, the amount of donor tissue available for transplantation is limited. Reconstructive surgery in which artificial ligament consisting of synthetic fibers was done. However, since cells are not adhered to the synthetic fibers, it was worn out with the lapse of time and is not used now.

Accordingly, the tissue engineering for tissues or organs of low self-repair potential are being vigorously studied in which autologous cells at healthy region are isolated, cultured to proliferate and differentiate, and then implanted again into same patients to regenerate them. Since isolated cells proliferate and differentiate by adhering on a surface of some substance, the development of excellent artificial extracellular matrix (referred to as "scaffold" hereinafter) is essential.

Generally, the conditions required for scaffolds are:
1) that it is not inflammatory and biocompatible;
2) that it is biodegradable;
3) that it is excellent for cell adhesion;
4) that it can maintain the cell activity; and
5) that it has three dimensional structure which enable tissues to regenerate by growth, migration and differentiation of cells.
   In addition, compressive stress up to about 20 MPa is applied to articular cartilage, and high tensile stress is applied to ligament and tendon. Accordingly, the scaffolds are further required
6) that it maintain shape stability after implantation; and
7) that it has mechanical strength.

From the view of tissue engineering, interstitial cells such as fibroblast, smooth muscle cell and endothelial cell are buried in a three dimensional framework (scaffolds) consisiting of biodegradable materials such as polyglycolic acid, cotton, cellulose, gelatin, collagen, polyhydroxyalkanoate, or non-biodegradable materials such as polyaramid, polyester, polystyren, polypropyren, polyacrylate, polyvinyl compound, polycarbonate, polytetrafuolroethylene, nitrocellulose, and cultured to prepare interstitial tissue which pack three dimensional structure crosslinked by interstitial cells and connective tissue protein which interstitial cells naturally secrets (Japanese Patent Kohyo 50611/1999).

As scaffolds of fibroblast for ligament or tendon repair with natural polymer, sponge or fiber of collagen (Dunn, M. G. et al., J. Biomed. Mater. Res., 29:1363-1671 (1955)), sponge-like structures in which glucosaminoglycan is bonded to collagen (Torres, D. S., et al., Biomaterials 21:1607-1619 (2000)), materials in which collagen is bonded to the surface of polylactic acid fiber (Ide,A., et al., Material Sci.Eng., C17:95-99 (2001)), or materials in which collagen is crosslinked with nordihydroguaseletinic acid (Koob, T. J. wt al., J. Biomed. Mater. Res., 56:40-48 (2001)) were used to examine the proliferation of fibroblast and the regeneration of ligament tissue.

However, in the method in which collage is used, since collagen scaffolds are allogenic, there is high probability that antigenic property and infection associated with it become serious problems. Furthermore, collagen gels and collagen materials do not offer the same flexibility for modification that might be achieved with biodegradable synthetic polymer. The lack of elasticity in collagen-based materials is an important problem as scaffolds of ligament or tendon.

Fiber or sponge in which polyglycolic acid or polylactic acid is used as biodegradable scaffolds for chondrocytes have been studied (Japanese Patent Kokoku No. 6155/1994; Japanese Patent Kohyo No. 511679/1996; Ito, K. et al., Mat Res. Soc. Proc., 252:359-365 (1992); Freed, L. E., et al., J. Biol. Mater. Res., 27:11-23 (1993)). These materials have advantages that they have no toxicity due to the identity of hydrolysis product thereof with physiological metabolite, have mechanical strength due to large polymer, and are easy to be molded. However, since these biodegradable synthetic polymers have not adhesiveness to cell, an attempt to increase cell adhesion by fixing biopolymer such as RGD (arginine-glysin-aspartic acid tripeptide) which is a cell adhesive factor in vivo, gelatin, and collagen on the surface of the material has been made (Yamaoka, T. et al., J. Biol. Macromol., 25:265-271 (1999)). However, such chemical fixing methods are laborious in manipulation, and possible residence of the reagents used for fixing reaction is concerned.

On the other hand, as a scaffold of chondrocyte using biopolymer, collagen gel or sponge-like materials (Japanese Patent Kokai No. 22744/1994; Japanese Patent Kohyo 510639/1999; Japanese Patent Kokai No.224678/2001; Fujisato, T., et al Biomaterial 21:153-159 (2000)) and sponge-like materials composed of chitin and chitosan were examined (Park, Y.J., et al., Biomaterial 21:153-159 (2000)). However these materials have problem in shape stability and mechanical strength. Collagen is expensive in material cost and infection with BSE is concerned.

Scaffolds consisting of a complex of acidic (anionic) and basic (cationic) polymers are reported (Japanese Patent kokai Nos. 335382/1994 and 277038/1994). The scaffold has a form of plate or film structure prepared by mixing both solutions and drying, and has not a three-dimensional porous structures composed of fibers. There are also described the method in which the above solution is coated or sprayed on an appropriate materials such as glass, metal or plastic. However, since the scaffolds of the whole are not biodegradable, they are not suitable as scaffolds for the regeneration of cartilage tissue in which biodegradability is required.

The method for preparing hybrid fiber consisting of biodegradable acidic polymer and basic polymer is reported (Japanese Patent kokai No 2002-291461). However, in the method for preparing the fiber, alginic acid is used as main material and a very small amount of basic polymer is added. The fiber is hydrophilic, swelled in medium and thus has not shape stability. The three dimensional scaffolds prepared by dissolving chitosan in acetic acid, spinning in calcium chloride solution, immersing in hyarulonic acid solution, reeling, drying, preparing fiber in a form of sheet, applying chitosan around the sheets and piling up the sheets are reported (Iwassaki et al., The 75th Japan Orthopedic Society Research Meeting, May 16 to 19, 2002 (okayama); Yamane et al., The 16th Japan Orthopedic Society Basic Research Meeting, October 18, 2001 (Hiroshima); Yamane et al., The 20th Japan Implantation and Regeneration Medicine Meeting, October 27, 2001 (Kyoto)). However, since the fibers of chitosan are made up as acetate salt, the fiber produced is gradually swelled or dissolved in medium, resulting in non-stability of shape and decreased growth of chondrocyte within the fiber.

### PROBLEM TO BE SOLVED BY THE INVENTION

It is an object of the present invention to provide scaffolds for animal cell which satisfies all the requirements above-mentioned. That is, it is an object of the present invention to provide scaffolds for animal cell having the following properties:
1) In the culture of animal cells, the seeding of cells is easy. Seeded or propagated animal cells can be easily adsorbed on and adhered to the scaffolds.
2) Animal cell such as chondrocyte and fibroblast propagate on the surface or inside of the scaffolds and secret extracellular matrix to form a connective tissue.
3) The scaffolds of the present invention have three dimensional space occupied by connective tissue formed.
4) The scaffolds of the present invention has sufficient mechanical strength till connective tissue is regenerated after implantion
5) The scaffolds of the present invention is biocompatible and biodegradable, and then, ultimately disappears after the regeneration of connective tissue.

### SUMMARY OF THE INVENTION

The present invention relates to a chitosan/biodegradable acidic biopolymer hybrid fibers in which the inner part of the fibers comprises chitosan or salts thereof and the outer part of the fibers is covered by a complex of chitosan and biodegradable acidic biopolymers and which retains the form thereof when the fibers are soaked in DMEM medium (Dulbecco's Modified Eagle's Medium) supplemented with 10 % FBS (fetal bovine serum) at room temperature for 2 weeks. The term "retain form" means has the original form without dissolving or swelling.

The present invention is also related to a method for preparing the fibers which comprise the steps of:
1) dissolving chitosan in an aqueous acid solution to prepare an aqueous solution of chitosan salt;
2) wet spinning the aqueous solution of chitosan salt using alkaline earth metal salts as coagulant to form fibers;
3) immersing the fibers in solution of biodegradable acidic biopolymers to react chitosan with acidic biopolymer on the surface of the fibers to form chitosan/acidic biopolymer hybrid fibers;
4) optionally stretching the hybrid fibers; and
5) treating the fibers with a base, a di- or more-basic inorganic acid or salt thereof, or a tri- or more-basic organic acid or salt thereof.

The present invention relates to a method for preparing the fibers which comprises the steps of:
1) dissolving chitosan in an aqueous acid solution to prepare an aqueous solution of chitosan salt;
2) wet spinning the aqueous solution of chitosan salt using a base, a di- or more-basic inorganic acid or salt thereof, or a tri- or more-basic organic acid or salt thereof as a coagulant to form fibers;
3) immersing the fibers in a solution of biodegradable acidic biopolymers to react chitosan with acidic biopolymers on the surface of the fibers to form chitosan/acidic biopolymer hybrid fibers; and
4) optionally stretching the hybrid fibers.

The present invention further relates to a three dimensional scaffolds for animal cells comprising the fibers. Animal cells include, but not be limited to, chondrocyte, fibroblast, nervous cell and undifferentiated cells which are subsequently differentiated into those cells.

The present invention relates to a method for culturing animal cells comprising culturing animal cells in vitro using the three dimensional scaffolds.

The present invention relates to a scaffold for grafts which are obtained by the above culture and in which proliferated animal cells are bonded to the scaffolds.

### BRIEF DESCRIPTION OF THE DRAWINGS

Figure 1 is a reproduction of an optical micrograph of chondrocyte after 21 days of culture using the three dimensional scaffolds of the present invention
Figure 2 is a reproduction showing the result of Alcian Blue Safranine staining of chondrocyte after 21 days of culture
Figure 3 is a graph showing the amount of protein and acidic mucopolysaccharide produced by chondrocyte per the three dimensional scaffold after 1, 7, 14 and 21 days of culture.

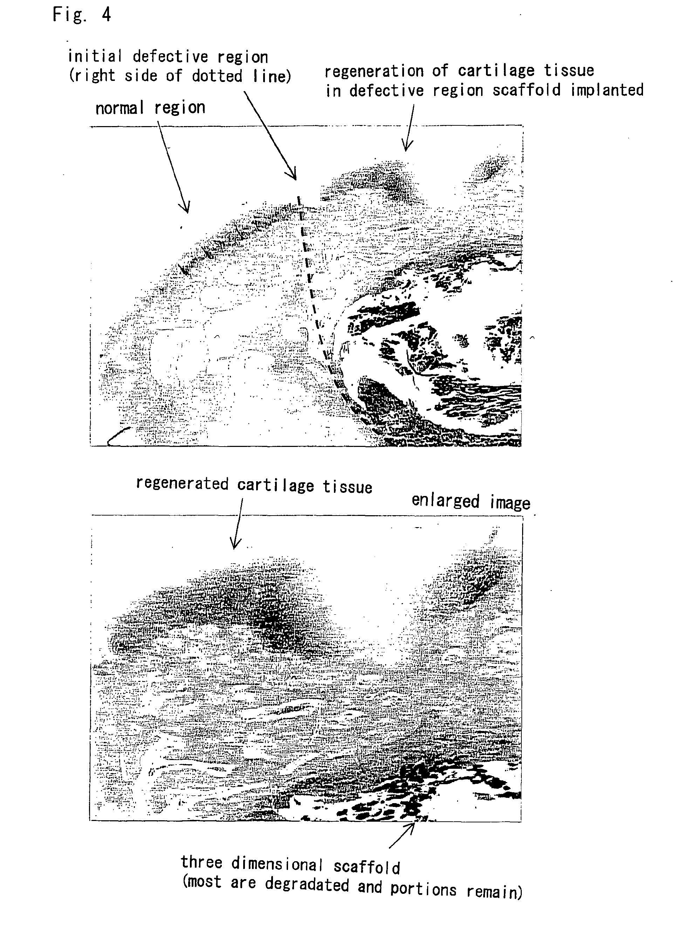

Figure 4 is a reproduction of optical micrograph of tissue stained with Safranin O. A defective portion on articular cartilage tissue of rabbit knee was prepared. To this place, rabbit chondrocytes cultured on the three dimensional scaffolds were implanted. On 8 weeks after the implantation, the defective portion was subjected to laparotomy and tissue observation was effected.
Figure 5 is a graph showing the amount of DNA of fibroblast per the three dimensional scaffold after 1, 7, 14 and 21 days of culture.
Figure 6 is a reproduction of a optical micrograph showing the result of type I collagen immunohistostaining by streptavidin-biotin method using anti-mouse antibody.
Figure 7 is a reproduction of a scanning electro microscope image of fibroblasts cultured for 28 days on three dimensional scaffolds.

### DETAILED DESCRIPTION OF THE PREFERRED EMBODIMENTS

The inventors contrived two methods for preparing chitosan/acidic biopolymer hybrid fibers in which the inner part of the fibers comprises chitosan or salts thereof and the outer part of the fibers is covered by a complex of chitosan and biodegradable acidic biopolymers and which retains the form thereof when the fibers are soaked in DMEM medium (Dulbecco's Modified Eagle's Medium) supplemented with 10% FBS at room temperature for 2 weeks.

In the first method, chitosan which is a basic polymer is dissolved in an aqueous acidic solution to prepare an aqueous solution of chitosan salt. Acids to be used may be inorganic acids or organic acids. Preferred examples of inorganic acids are monobasic acids such as hydrogen chloride and nitric acid. Preferred examples of organic acids are formic acid, acetic acid, propionic acid, butyric acid, ascorbic acid, and the like.

The aqueous solution of chitosan salt is subjected to wet spinning to form fibers. "Wet spinning" is a method in which spinning solution of polymer is extruded through a nozzle into a coagulating bath having high ability to desolvate to coagulate the polymer.

Water soluble salt of alkaline earth metals such as calcium, magnesium, barium, for example, halogen salts, can be used as a coagulating agent. Calcium chloride is most preferred. The alkaline earth metal salt is dissolved in water, or a mixture solvent of water/methanol and used. The concentration of the alkaline earth metal salt is from 10% to a saturated concentration, preferably from 40% to 60%.

The chitosan salt solution is extruded through a nozzle into a coagulating bath containing the coagulating agent to coagulate chitosan to form fibers. The fibers formed are immersed into a mixture solvent of water-miscible solvent and water to remove excessive coagulant.

The term "acidic biopolymers" means polymers derived from natural source having acidic groups such as carboxyl group, sulfate group, sulfonate group, phosphate group, or salt thereof. In a preferred embodiment, the biopolymers are polysaccharides. "Acidic biopolymers" also include molecular weight decreased naturally occurring biopolymer or acidic groups formed naturally occurring biopolymer by any physical, chemical or enzymatic means.

Examples of the acidic biopolymers containing carboxyl groups include polymers containing gluconic acid, glucuronic acid, iduronic acid, D-mannuronic acid, galactruronic acid, gluronic acid, sialic acid, and glutamic acid, for example, hyaluronic acid, alginic acid, heparin, or polyglutamate.

Examples of the acidic biopolymers containing sulfate groups include chondroitin sulfate, dermatan sulfate, heparin, heparan sulfate, keratan sulfate. Examples of the acidic biopolymers containing phosphate groups include DNA and RNA. Two or more kinds of these acidic biopolymers may be used for the preparation of a complex of chitosan and acidic biopolymers.

The complex of chitosan and acidic biopolymers means a complex formed by electrostatic interaction between cationic group of chitosan and anionic group of acidic biopolymers. Chitosan may be contacted with acidic biopolymers in an appropriate medium, for example, water, to form the complex. It was found that for the formation of the complex, it is not necessary to combine both the aqueous solutions. Solid chitosan may be contacted with acidic biopolymer solutions.

Solution in which an acidic biopolymer is dissolved, for example, in water or water/alcohol is prepared. The concentration of acidic biopolymer is 0.01 % to 10 %, preferably 0.05% to 1 %. When the chitosan fibers above are immersed in the acidic biopolymer solution, a complex of chitosan (basic polymer) and acidic biopolymer is formed on the surface of chitosan fibers. The resulting fibers are hybrid fibers in which the inner portion comprises chitosan and the outer surface is coated with the complex of chitosan (basic polymer) and acidic biopolymer. The fibers may be optionally extruded. The formation of the complex may be effected after extrusion.

The thus resulting fibers are after-treated with an aqueous solution of inorganic base, di or more-basic inorganic acid or salt thereof, or tri- or more-basic organic acid or salt thereof, or a solution thereof using mixture solvent of water and water miscible organic solvent. Without the after-treatment, the fibers may be gradually dissolved in culturing medium and thus has not shape stability as a scaffold. The examples of inorganic bases include alkaline metal hydroxide such as NaOH and KOH. Examples of di- or more-basic inorgan acids include sulfuric acids and phosphoric acids. Examples of salts thereof include sodium carbonate, sodium bicarbonate, trisodium phosphate, sodium dihydrogenphosphate, disodium hydrogenphosphate, sodium sulfate, sodium hydrogensulfate and potassium salts and ammonium salta corresponding to such sodium salt. Examples of tri- or more-basic organic acids include citric acid, ethylenediamine tetraacetic acid, 1,1,2-tricaboxyethane, 1,1,2-tricaboxy-2-methylethane, 1,1,3-tricaboxypropane, 1,2,3-tricaboxypropane, 1,1,2,2-tertracaboxyethane, and 1,2,2,3-trtracaboxyepropane. The concentration of these after-treatment agents is 0.1 to 10 %. It is preferably 0.2 to 1 % for alkaline metal hydroxides and 1 to 3 % for inorganic acids and organic acids. After the treatment, the fibers are thoroughly washed with water and immersed in methanol to dehydrate and dry them.

The second method for preparation of the fibers is different from the' first method for preparation of the fibers in that inorganic base, di- or more-basic inorganic acids or salts thereof, or tri- or more-basic organic acids or salts thereof is used as a coagulant, and that the after-treatment is not effected. That is, chitosan is dissolved in acidic aqueous solution to form an aqueous solution of chitosan salt. The aqueous solution is subjected to wet spinning using inorganic bases, di- or more- basic inorganic acids or salts thereof, or tri- or more-basic organic acids or salts thereof as a coagulant to form fibers. The resulting fibers are then immersed in a mixture solvent of water-miscible organic solvent and water to remove excessive coagulants. The fibers are immersed in a solution of biodegradable acidic biopolymers to react chitosan with acidic biopolymer on the surface of the fibers to form chitosan/acidic biopolymer hybrid fibers. The fibers may be optionally extruded. Examples of inorganic bases, di-or more-basic inorganic acids or salts thereof, or tri- or more basic organic acids or salts thereof as a coagulant are the same as mentioned as the after-treatment agents in the first method for the hybrid fibers preparation above. The concentration of the coagulant is in the range of 0.5 % to 30%. The concentration of chitosan, the kind of acid necessary to disolve chitosan, the concentration of acidic biopolymers may be the same as in the first method for preparation.

The chitosan/acidic biopolymer hybrid fibers thus prepared in which the inner portion of the fibers comprises chitosan and the surface is covered with the complex of chitosan and acidic polymers has considerable mechanical strength, can be adhered to animal cell such as chondrocyte and fibroblast, and is biodegradable and biocompatible.

In order to use as implanting scaffold for animal cells, the scaffolds must be a three dimensional structure having a space in which cell can proliferate and which retain extracellular matrixes secreted from the cells. The three dimensional scaffolds must also have form-stability and mechanical strength when strength is applied to them. The three dimensional scaffolds which satisfy the above requirement can be produced from the texture, knitting or interlace prepared from the hybrid fibers prepared by the method of the present invention. The texture, knitting or interlace can be produced by conventional methods. The texture, knitting or interlace may be folded or piled as necessary to give thickness. The texture, knitting or interlace which is folded or piled is unified using the hybrid fibers of the present invention. The texture, knitting or interlace folded or piled may contain the fiber in the inside in a form other than these forms. The form of the three dimensional scaffolds may be changed in accordance with injury portion.

The three dimensional scaffolds of the present invention thus have the following advantageous properties as scaffolds.
1) In the culture of animal cells such as chondrocyte and fibroblast, seeding of cell is easy. Seeded or proliferated animal cells such as chondrocyte and fibroblast can be adsorbed on or adhered to scaffolds.
2) Animal cells such as chondrocyte and fibroblast proliferate on the surface or inside of the scaffolds and secret extracellular matrixes to form a connective tissue.
3) The scaffolds of the present invention have three dimensional space which can be occupied by formed connective tissue.
4) The scaffolds of the present invention have sufficient mechanical strength till connective tissue is regenerated after implantation
5) The scaffolds of the present invention are biocompatible and biodegradable and ultimately disappears after the regeneration of the tissue.

The culture of animal cells using the three dimensional scaffolds of the present invention can be effected pursuant to conventional method for culturing animal cell (see, for example, Klagsburn, M., "Large Scale Preparation of Chondrocytes", Methods in Enzymol., 58:560 (1979)). First of all, the scaffolds are subjected to disinfection treatment by heating in an autoclave or by gas in such a manner that the forms and properties of the scaffolds are not disrupted. Animal cells are then seeded as evenly as possible on the three dimensional scaffolds and cultured. Cells used for culture may be derived from mammal such as rabbit, cattle, horse, dog, cat, and human cell. Preferred are human cells. Most preferred are cells from patient which undergos implantation.

Medium used in conventional animal cell culture, for example, DMEM (Dulbecco's Modified Eagle's Medium) containing human serum can be used. Any growth factor such as TGF-beta (transforming growth factor-beta), FGF (fibroblast growth factor), ChM-1 (chondromojelin-1) may be added to the medium.

In order for seeded cells to proliferate and differentiate on scaffolds, it is crucial to have high cell adhesion and adsorption.

Since cartilage tissue is under low oxygen condition due to lack of blood vessel in it and suffers pressure loading due to body weight, the culture under the condition similar to these physiological condition may be effective. Accordingly, it is possible to effect the culture under low oxygen concentration of 1 to 15 % ,or under pressure of 0.1 to 20 MPa (0.01 to 2 Hz in the case of cyclic applying), or under combined condition of these conditions. Specifically, the method for applying pressure include a method for applying air pressure or water pressure to the medium using a pump or a piston.

Since stretching stress is applied to ligament and tendon, it might be effective to culture under the condition similar to physiological stretching conditions. Accordingly, in the culture of fibroblast, culture may be effected under a stretching stimulus of 0.01 to 50 mm/cm (0.01 to 2 Hz in the case of cyclic applying). Specifically, applying of stretching stress is effected by adding stretch and contraction change while both ends of the scaffolds immersed in medium are fixed to an instrument having an ability to stretch and contract.

Culture of chondrocyte is at least continued till extracellular matrixes are formed. Usually, by 2 to 4 weeks, chondrocytes are adhered on the surface of three dimensional scaffolds of the present invention, proliferate and secret extracellular collagen-like matrix.

The three dimensional scaffolds thus prepared comprising hybrid fibers of chitosan and acidic biopolymers and the scaffolds to which cartilage tissue is adhered can be used as a graft for repairing cartilage damage.

Culture of fibroblast is at least continued till extracellular matrixes are formed. Usually by 2 to 4 weeks, fibroblast is adhered on the surface of the three dimensional scaffolds of the present invention, proliferate and secret extracellular collagen-like matrix. Culture may be optionally effected for about 2 months to prepare sufficient tissue for implantation in vitro.

The three dimensional scaffolds thus prepared comprising hybrid fibers of chitosan and acidic biopolymers and the scaffolds to which fibroblast is adhered can be suitably used as a graft for repair of ligament and tendon damage.

In the case of undifferentiated cells, for example, mesenchyme stem cells are isolated from bone marrow liquid by a density-gradient centrifugation. It is possible to add growth factors such as TGF-beta and FGF to a medium such as DMEM to differentiate the stem cell to chondrocyte or fibroblast before seeding the cells on the three dimensional scaffolds, proliferating and differentiating them. Alternatively undifferentiated cells may be seeded on the scaffolds.

It is possible to add EGF (epidermal growth factor) to a medium using nervous stem cell to differentiate it to nervous cell. Alternatively, seeding of the cells on the scaffolds may be effected in undifferentiated cells.

The prestnt invention will be illustrated by referring to examples. However, the present invention is never limited to the examples

### EXAMPLES

### Example 1

### Preparation of chitosan and hyaluronic acid hybrid fiber (1) and (2)

A solution in which 8(w/v)% chitosan (Kimitu Chemical Industry, F2P, Molecular weight: about 165,000) was dissolved in 4% aqueous acetic acid solution was packed with a column (glass, 45 mm in inner diameter, 45 mm in length) and filtered through a filtering cloth under compression (0.6kgf/cm²). The filtrate was packed with a spinning column (glass, inner diameter 45 mm, length 410 mm) and used as a spinning liquid to prepare fibers with a simple spinning apparatus in the following method: The spinning solution above was extruded through a nozzle having 50 holes (φ 0.1 mm) under a compression of 0.8 kgf/cm² into saturated calcium chloride aqueous solution (the first coagulating bath: water/methanol=1/1 (by volume); 100cm in bath length; about 2L in volume) to form fibers. The fibers were then immersed in water/methanol =1/1 by volume (the second coagulating bath: 50 cm in bath length, about 1 L in volume), further immersed in 0.05 % hyaluronic acid solution (water/methanol=1/1 (by volume)) (the third bath: 50 cm in bath length, about 150 mL in volume). The fibers were then passed through rollers (first roller, speed 3.2 m/min; second roller, speed 3.2 m/min; draw ratio 1.0) and winded up with a wind-up roller. The fibers were immersed in 0.8(w/v)% sodium hydroxide solution (water/methanol=1/9 (by volume)) for 15 hours, then washed with water, further immersed in methanol for about 2 hours and air dried at room temperature, or removed from the roller and air dried at a room temperature to give soft and tender chitosan-hyaluronic acid hybrid fibers (referred to as "chitosan-hyaluronic acid hybrid fiber (1)" hereinafter)

Another fibers were prepareed in the similar method except that the concentration of hyarulonic acid is 0.1 (w/v) % to give a soft and tender chitosan-hyaluronic acid hybrid fibers (referred to as "chitosan-hyaluronic acid hybrid fiber (2)" hereinafter).

### Comparative Example 1

### Preparation of hybrid fiber without after-treatment

Fibers were prepared in the same condition as chitosan-hyaluronic acid hybrid fiber (1) except that the after-treatment with sodium hydroxide solution after the spinning was not effected.

### Comparative Example 2

### Preparation of chitosan (alone) fiber (a)

Chitosan (alone) fibers were prepared in the similar way as Example 1 except that hyaluronic acid is not added in the third bath (referred to as "chitosan fiber (a)" hereinafter).

### Example 2

### Preparation of chitosan and hyaluronic acid hybrid fiber (3) and (4)

A solution in which 3.5(w/v)% chitosan (Kimitu Chemical Industry, B, Molecular weight: about 600,000) was dissolved in 2 % acetic acid aqueous solution was packed with a column and filtered through a filtering cloth under compression. The filtrate was packed with a spinning column and used as spinning liquid to prepare fibers using a simple spinning apparatus in the following method: The spinning solution above was extruded through a nozzle having 50 holes (φ 0.1 mm) under a compression of 0.8 kgf/cm² into 53 (w/v) % calcium chloride solution (the first coagulating bath: water/methanol=1/1 (by volume), 100 cm in bath length, about 2L in volume) to form fibers. The fibers were then immersed in water/methanol =1/1 by volume (the second coagulating bath: 50 cm in bath length, about 1L in volume), further immersed in 0.05 % hyaluronic acid solution (water/methanol=1/1 (by volume)) (the third bath: 50 cm in bath length, about 150mL in volume). The fibers were then passed through rollers (first roller, speed 4.4 m/min; second roller, speed 4.5 m/min; draw ratio 1.02) and winded up with a wind-up roller. The fibers were immersed in 0.2% sodium hydroxide solution (water/methanol=1/9 (by volume)) for 15 hours, then washed with water, further immersed in methanol for about 2 hours and air dried at room temperature, or removed from the roller and air dried at room temperature to give soft and tender chitosan-hyaluronic acid hybrid fibers (referred to as "chitosan-hyaluronic acid hybrid fiber (3)" hereinafter)

Another fibers were prepared in the similar method except that the concentration of hyarulonic acid is 0.1 % to give soft and tender chitosan-hyaluronic acid hybrid fibers (referred to as "chitosan-hyaluronic acid hybrid fiber (4)" hereinafter).

### Comparative Example 3

### Preparation of chitosan (alone) fiber (b)

Chitosan (alone) fibers were prepared in the similar way as Example 2 except that hyaluronic acid is not added in the third bath (referred to as "chitosan fiber (b)" hereinafter).

### Example 3

### Preparation of hybrid fibers using various after-treating agents

Chitosan-hyarulonic acid hybrid fibers were prepared in the similar methods as in Example 2 (hyarulonic acid concentration 0.05%) except that the following after-treating agents are used in place of 0.2% (w/v) sodium hydroxide solution (water/methanol=1/9 (by volume)).

**Table 1**

| Aftertreating agent | Concn. | Solvent composition |
|---|---|---|
| Potassium Carbonate | 2% | water/methanol=1/9 (volume) |
| Sodium Carbonate | 2% | water/methanol=1/1 " |
| Sodium bicarbonate | 1% | water/methanol=1/1 " |
| Sodium bicarbonate | 3% | water/methanol=3/2 " |
| Tripotassium Phosphate | 2% | water/methanol=1/1 " |
| Dipotassium hydrogen phospate | 2% | water/methanol=1/1 " |
| Sodium dihydrogen phospate | 2% | water/methanol=1/1 " |
| Sodium sulfate | 2% | water/methanol=3/2 " |
| Citric Acid | 3% | water/methanol=1/9 " |

### Example 4

### Preparation of hybrid fiber using various coagulants

A solution in which 3.5(w/v)% chitosan (Kimitu Chemical Industry, B, Molecular weight: about ·600,000) was dissolved in 2 % acetic acid aqueous solution) was packed with a column and filtered through a filtering cloth under compression. The filtrate was packed with a spinning column and used as a spinning liquid to prepare fibers using a simple spinning apparatus in the following method: The spinning solution above was extruded through a nozzle having 50 holes (φ 0.1 mm) under a compression of 0.8 kgf/cm² into the various coagulating liquids shown in Table 2 (first coagulating bath: 100 cm in bath length, about 2L in volume) to form fibers. The fibers were then immersed in water/methanol =1/1 by volume (second coagulating bath: 50 cm in bath length, about 1L in volume),further immersed in 0.05 % hyaluronic acid solution (water/methanol=1/1 (by volume)) (50 cm in bath length, about 150mL in volume). The fibers were passed through rollers (first roller, speed 4.4 m/min; second roller, speed 4.5 m/min; draw ratio 1.02) and winded up with a wind-up roller. The fibers were immersed in water for 10 minutes, washed again with water, further immersed in methanol for about 2 hours, followed by drying to give fibers.

**Table 2**

| Coagulant | Concn. | Solvent composition |
|---|---|---|
| | | |
| Sodium hydroxide | 5% | water/methanol=1/1 (volume) |
| Sodium carbonate | 5% | water/methanol=1/1 (volume) |
| Tripotasium phosphaate | 5% | water/methanol=1/1 (volume) |
| Sodium sulfate | 5% | water/methanol=1/1 (volume) |

### Example 5

### Preparation of chitosan and alginic acid hybrid fiber

Chitosan and alginic acid hybrid fibers were prepared in the similar way as in Example 2 except that 0.05 % and 0.1 % alginic acid were used respectively in place of 0.05 % hyaluronic acid

### Example 6

### Tensile strength of chitosan and hyaluronic acid hybrid fiber

Tensile strength and elongation of each fiber prepared in Examples 1 and 2 were measured. The load at break and elongation were measured in accordance with JIS standard L1015. The sectional area of each fibers were measured by image processing under a microscope.

### Method for measuring breaking force and strain:

Each fiber in the form of thread (prepared by binding 50 monofilaments) was cut to about 40 mm in length, both the ends thereof were put in the adhesive paper (for example, using post-it) to prepare each sample in which the distance between gages is 20 mm. The both ends of the sample are fixed to clip-type gripping tools (product number 343-06742-03, Shimadzu Corporation, Japan) and the upper gripping tool is suspended to a load cell(20 N, product number 346-51294-03, Shimadzu Corporation, Japan) before it was set to a desk-type material testing machine (AGS-H, product number 346-51299-02, Shimadzu Corporation, Japan). It was vertically pulled in a pulling rate of 20 mm/min and breaking force and strain were measured from the force and displacement at failure. These data are incorporated in a personal conputer (IBM, NetVista A 40). A software (TRAPEZIUM, Shimadzu Corporation, Japan) was used for measurement and analysis of data.

### Method for measuring sectional area of fiber:

Each fiber in the form of thread (prepared by binding 50 monofilaments) was cut to about 5 mm in length, inserted into holes in a plastic plate having small apertures of diameter of about 1 mm and fixed to it. The plastic plate was placed on the dais of an optical microscope (BX50, Olympus Optical Industry). The image of fiber section was caught through a camera control unit (ICD-740, Ikegami Tusinki) containing a CCD camera. The sectional area of the fibers were determined by an image processing instrument (VIDEO MICRO METER, Model VM-30, Olympus Optical Industry, Monitor Screen TM150, Ikegami Tusinki). The strength and strain at failure of each fiber are shown in Table 3.

**Table 3**

| Strength and Strain of chitosan and hyarulonic acid hybrid fiber (1) and (2) (Averaged value±standard error, n=5) | | | | |
|---|---|---|---|---|
| Fiber | Maximum breaking force (N) | Sectional Area (µm²) | Strength (N/mm²) | Strain at failure (%) |
| Chitosan Fiber (a) | 1.56±0.06 | 12199.64 ±205.47 | 128.07 ±4.87 | 4.26 ±0.41 |
| | | | | |
| Chitosan- | 2.35±0.08 | 15397.20 | 152.61 | 6.11 |
| hyarulonic acid hybrid fiber(1) | | ±187.03 | ±5.39 | ±0.72 |
| Chitosan-hyarulonic acid hybrid fiber(2) | 4.10±0.16 | 18843.20 ±284.69 | 217.55 ±8.47 | 3.14 ±0.28 |

Although the strength of chitosan (alone) fiber is about 130 N/mm², hybridization with hyaluronic acid increases it to about 150-220 N/mm². A fiber in which collagen fiber is crosslinked with nordihydroguayaletinic acid has a strength of 50 N/mm² (Koob, T.J., Et al., J. Biomed. Mater Res., 56:40-48 (2001). Accordingly, it was confirmed that chitosan-hyarulonic acid hybrid fiber had about three- to five-fold strength compared with collagen fiber

**Table 4**

| Strength and Strain of chitosan and hyarulonic acid hybrid fiber (3) and (4) (Averaged value±standard error, n=5) | | | | |
|---|---|---|---|---|
| Fiber | Maximum breaking force (N) | Sectional Area (µm²) | Strength (N/mm²) | Strain at failure (%) |
| Chitosan Fiber (b) | 2.86±0.03 | 15576.08 ±332.20 | 183.60 ±1.65 | 2.54 ±0.16 |
| Chitosan-hyarulonic acid hybrid fiber(3) | 4.17±0.08 | 19165.34 ±133.35 | 217.60 ±3.93 | 5.89 ±0.26 |
| Chitosan-hyarulonic acid hybrid fiber(4) | 3.58±0.06 | 18031.82 ±243.88 | 198.51 ±3.47 | 4.55 ±0.22 |

### Example 7

### Adhesion of chondrocyte on chitosan and hyarulonic acid hybrid fiber

In order for chondrocyte to proliferate and differentiate on a scaffold, it is necessary for chondrocytes to adhere to a scaffold as much as possible. The adhesion of chitosan (alone) fiber and chitosan-hyaluronic acid hybrid fiber prepared in Example 2 to chondrocytes was determined. Commercially available degradable seaming thread for medical use (biodegradable synthetic polymer, polyglactin-910, Vicryl3-0, Ethicon Co., NJ, USA) was used as a control.

Chondrocytes were isolated from the articular surfaces of a Japanese white rabbit (8 weeks age, body weight 1.8-2.0 kg). The concentration of the cell was 2x10⁶ cells/ml. The evaluation of adhesion was in accordance with a method of Nishimura (NIshimura, J. Biol. Macromol, 7:100-104, 1985). That is, each fibers were cut to 10 mm in length and a fixed amount of the fiber (100 mg) was packed into a teflon tube (5 mm in inner diameter, 30 mm in length). One hundred microliter of the suspension containing chondrocytes was loaded onto one end of the tube and incubated at 37 °C for 1 hour. One mililitter of PBS (phosphate buffered saline) was then passed through the tube to give an eluted solution. The number of cells in the effluent was counted and the ratio of cell effluence was calculated..

**Table 5**

| Comparison of chondrocyte adhesion to various fibers | |
|---|---|
| Sample | Percent effluence of cell (average±standard error, n=5) |
| Vicryl | 64.2±7.5 |
| chitosan fiber | 13.0±2.5* |
| chitosan-hyarulonic acid fiber (3) | 11.1±6.4* |
| chitosan-hyarulonic acid fiber (4) | 26.9±6.6* |

| | |
|---|---|
| *p<0.05 vs Vicryl | |

As shown above, cell adhesion between Vicryl and the fibers of the present invention is significantly different in ANOVA statistical analysis.

### Example 8

### Adhesion of fibroblast on chitosan and hyarulonic acid hybrid fiber

In order for fibroblast to proliferate and differentiate on a scaffold, it is necessary for fibroblast to adhere to the three dimensional scaffold as much as possible. The adhesion of chitosan (alone) fiber and chitosan-hyaluronic acid hybrid fiber prepared in Example 1 to fibroblast was determined. Commercially available degradable seaming thread for medical use (biodegradable synthetic polymer, polyglactin-910, Vicryl, Ethicon Co., NJ, USA) was used as control. Test Method:

Fibroblasts were isolated from the pateller tendon of a Japanese white rabbit (8 weeks age, body weight 1.8-2.0 kg). The concentration of the fibroblast was 1x10⁷ cells/ml. The evaluation of the adhesion was in accordance with the method of Nishimura (Nishimura, J. Biol. Macromole, 7:100-104, 1985). That is, each fibers were cut to 10 mm in length and a fixed amount of the fibers were packed into a Teflon (Trade Mark) tube (5mm in inner diameter, 30 mm in length). One milliliter of the solution containing fibroblasts was loaded onto one end of the tube and incubated at room temperature for 15 minutes. One milliliter of PBS (phosphate buffered saline) was then passed through the tube to give an eluted solution. The number of cells in the effluent was counted and is considered to be the number of cell which did not adhere to the fiber.

**Table 6**

| Comparison of fibrobladst adhesion to various fibers | |
|---|---|
| Sample | number of effluent fibroblast (average±standard error, n=5) |
| Vicryl | 151.1±4.8 |
| Chitosan fiber | 44.4±7.2* |
| Chitosan-hyarulonic acid fiber (1) | 12.0±3.1*'** |
| Chitosan-hyarulonic acid fiber (2) | 14.8±7.7*'** |

| | |
|---|---|
| *p<0.05 vs Vicryl | |
| ** p<0.05 vs chitosan fiber | |

As shown above, there is significant difference in ANOVA statistical analysis in cell adhesion between Vicryl and the fibers of biopolymers. Statistically significant difference is observed between chitosan (alone) fiber and the chitosan-hyarulonic acid hybrid fiber, demonstrating that the chitosan-hyarulonic acid hybrid fiber has better adhesion to fibroblast

### Example 9

### Shape stability of fiber in medium

About 20 mg of each of the chitisan-hyaluronic hybrid fiber prepared in Examples 1 - 4, the chitisan-hyaluronic hybrid fiber prepared in Comparative Examples 1, the chitosan (alone) fiber prepared in Comparative Examples 2 and 3 was placed in a tube, respectively, and 2 ml of DMEM (Dulbecco's Modified Eagle's Medium, Sigma, Code D5796) supplemented with 10% FBS (fetal bovine serum) was added to it and was left at room temperature for two weeks.

The hybrid fiber prepared in Comparative Example 1 in which calcium chloride was used as coagulating agent and the after-treatment was not effected became unclean in shape and the color of medium was changed to yellow. In other fibers, shape change was not observed and the color of medium remains red.

### Example 10

### Preparation of three dimensional scaffolds from chitosan and hyaluronic acid hybrid long fiber

Long fibers of more than 100 m in length was prepared from the fibers in which spinning were effected in the method described in Example 1 followed by the treatment sodium hydroxide and methanol. After the long fibers are subjected to twisting to form twisted yarn, a zonary structure was prepared from the yarn using commercially available braider. The three dimensional scaffolds having a fixed shape were prepared using it.

### Example 11

### Culture of chondrocyte using three dimensional scaffold

Culture test was effected using-the three dimensional scaffolds prepared in Example 10. Chondrocytes were collected and cultured in accordance with methods of Kawasaki (Kawasaki, K., et al., J. Cell Physiol., 179:142-148 (1999) and Yasui (Yasui, N., et al., Exp. Cell Biol., 50:92-100(1982)). That is, a piece of cartilage tissue was isolated from the articular surfaces of a Japanese white rabbit (8 weeks age, body weight 1.8-2.0 kg) and treated at 37°C for 25 minutes by adding 0.25 % trypsin solution before treating at 37°C for 25 minutes by adding 0.25 % collagenase (Type II) solution to isolate cells. After 50 µl of this cell suspension was removed, 50 µl of Trypan blue was added, the suspension was thoroughly stirred. Twenty microliters of the suspension were placed on hemocytometer and the number of the cells was counted to calculate the total number of the cells.

The three dimensional scaffolds of the chitosan and hyarulonic hybrid fibers which have been previously sterilizd in an autoclave were placed in a multi-well plate (12 wells, Falcon). One hundred microlitter of a suspention containing chondrocytes were added on each scaffold in well so that each well contained 5x10⁵ chondrocytes cells. After incubating under 5 % CO₂ in an incubator at 37°C for 1 hour, 2 ml of DMEM medium was added by portion and 20 µl of 0.1% ascorbic acid phosphate was further added and the mixture was incubated under the above conditions.

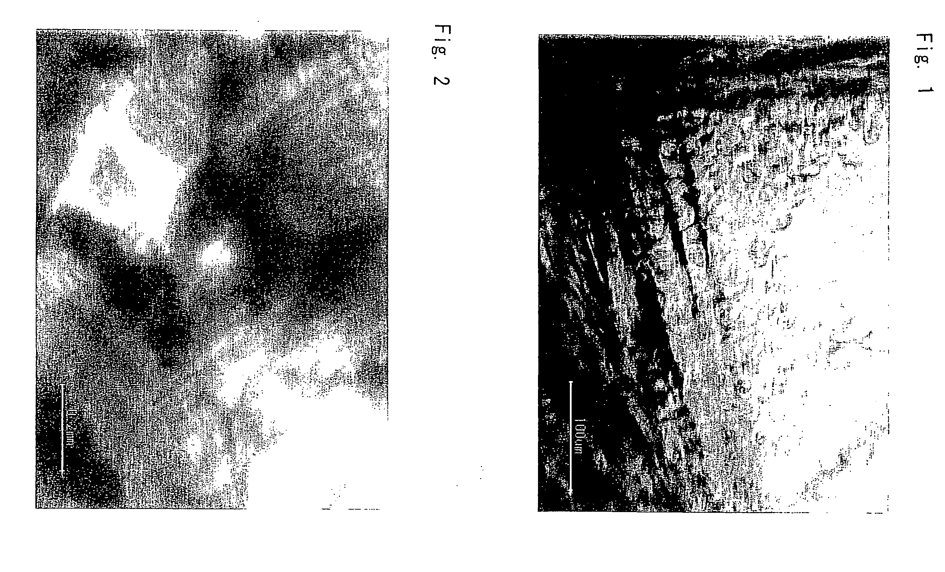

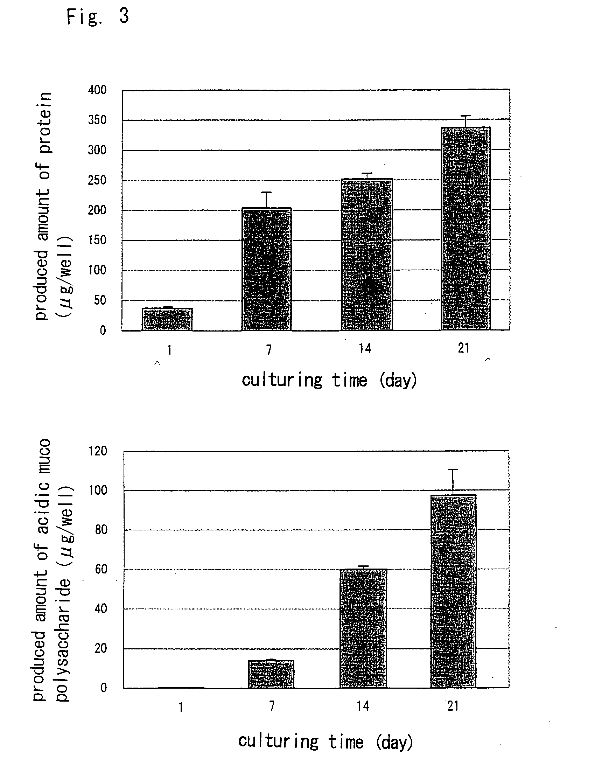

Figure 1 shows an optical micrograph of chondrocytes after 21 days of culture. It is confirmed that seeded chondrocytes proliferate well on the fiber and in aperture between the fibers. Figure 2 shows the result of Arcyanblue Safranin staining which confirm many extracellular matrixes stained strongly in blue. It is known that the chondrocytes are favorably proliferate and differentiate on the fiber and secret extracellular matrixes such as chondroitin sulfate. Figure 3 shows an produced amount of proteins and acid mucopolysaccharides per the three dimensional scaffold after 1, 7, 14 and 21 days of culture. The amount of produced proteins and acid mucopolysaccharides increased with culture. From these facts, it is known that good proliferation and differentiation of chondrocyte produce various proteins and extracellular matrixes such as chondroitin sulfate.

### Example 12

### Evaluation of cartilage tissue regeneration in implantation of cultured three dimensional scaffold into animal

A defective portion of about 4 x 6 x 1.5 mm on articular cartilage tissue of the knee was prepared under anesthesia in Japanese white rabbit (8 weeks age, body weight 1.8-2.0 kg). To this place, the three dimensional scaffold of Example 10 on which chondrocytes of rabbit had been cultured for two weeks was implanted and both the sides of the scaffold were lightly fixed with a seaming thread. On 8 weeks after the implantation, the defective portion was subjected to laparotomy and tissue observation was effected with Safranin O staining. Cartilage tissue which was stained in red was observed in the defective portion to which the three dimensional scaffold was implanted, confirming that cartilage tissue was regenerated. Fixation of the scaffold to the cell was also good and it was observed that the scaffold has been gradually decomposed. Extravastion of inflammatory cell associated with the scaffold implantation was scarcely observed. Accordingly, it is considered that no strong immune response associated with the scaffold implantation occurred.

### Example 13

### Culture of fibroblast using three dimensional scaffold

Fibroblasts were collected and cultured in accordance with Matin's method (Martin, G. M., Tissue Culture, Methods and Application, Academic Press, 39, 1973). A piece of 2mm x 2mm was prepared from the patellar tendon of a Japanese white rabbit (8 weeks age, body weight 1.8-2.0 kg) and covered with a cover glass and fixed to a dish having a diameter of 35 mm. To this, DMEM supplemented with 10% FBS was added and the mixture was incubated under 5% CO₂ in a incubator at 37°C for two weeks. When fibroblasts became confluent, medium was removed and fibroblasts were washed with PBS (-). 0.5ml of 0.25 % trypsin was added and incubation was effected at 37°C for 15 minutes, followed by the addition of 1 ml of the medium to obtain cell suspension. To this suspension, 50 µl of 0.04 % Trypan Blue were added and the number of the cells was counted using a hemocytometer.

The three dimensional scaffolds of chitosan and hyarulonic hybrid fiber which had been previously sterilized in an autoclave were placed in a plate (12 holes). One hundred microliters of solution containing fibroblasts were added on each scaffold in well so that each well contained 1x10⁵ cells. After incubating under 5 % CO₂ in an incubator at 37°C for 1 hour, 2 ml of DMEM medium was added and the mixture was incubated under the above conditions. The amount of DNA was determined after 1, 7, 14 and 21 days of culture as an index of cell number.

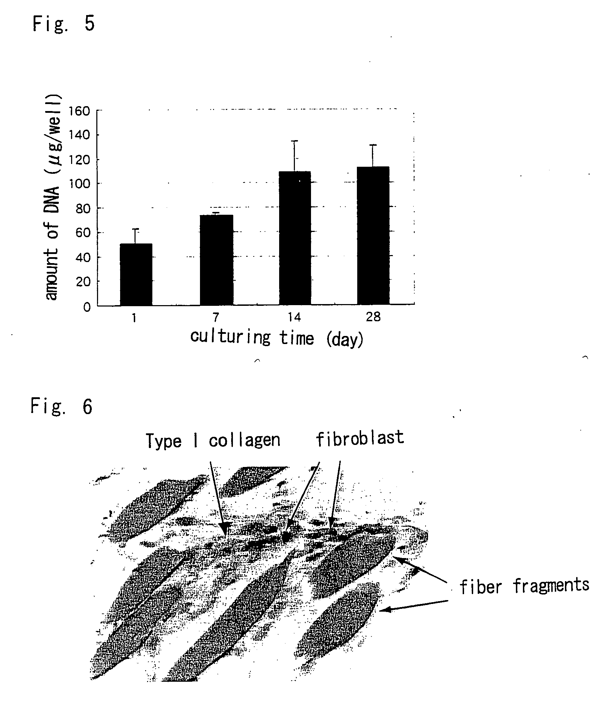

The measurement was effected in accordance with Rago's method (Rago, R. et al., Anal. Biochem., 191:31-34 (1990)). That is, sodium chloride was added to 0.05 M phosphate buffer (pH 7.4) to make 2M solution of sodium chloride. Cultured fibroblasts were removed together with the scaffold and the medium was washed with PBS. The scaffolds are finely cut with scissors before transferring it into a 1.5 ml tube. One mililiter of 0.05 M phosphate buffer (containing 2 M sodium chloride, pH 7.4) was added to the tube, stirred thoroughly and left at room temperature for 1 minute. After the solution was enough stirred again, the supernatant thereof was used as a sample solution. To 100 µl of the sample solution, 2 ml of 0.05 M phosphate buffer (containing 2 M sodium chloride, pH 7.4) was added, followed by the addition of 10 µl of a fluorescent reagent (Hoechst 33258, 0.02% solution was prepared using purified water). After the solution was thoroughly stirred, the intensity of fluorescence of the resulting solution was measured with a spectrofluorometer (RT-5300PC, Shimadzu Corporation, Japan)(excitation at 356 nm; emission at 458 nm). A calibration curve was made using standard DNA solution in the range of 0-125 µg/mL. The DNA concentration contained in the sample solution was determined from the calibration curve. As shown in Figure 5, the amount of DNA generated increase with culturing, confirming that fibroblasts benignly proliferate on the three dimensional scaffold.

Figure 6 shows the result of immunohistochemicalstaining of type I collagen (cultured on the three dimensional scaffold for 28 days) by the streptavidin-biotin method. Eextracellular substrate was stained and it was demonstrated that the three dimensional scaffold prepared from the hybrid fibers of the present invention was excellent in the production of type I collagen which is an extracellular matrix of ligament and tendon.

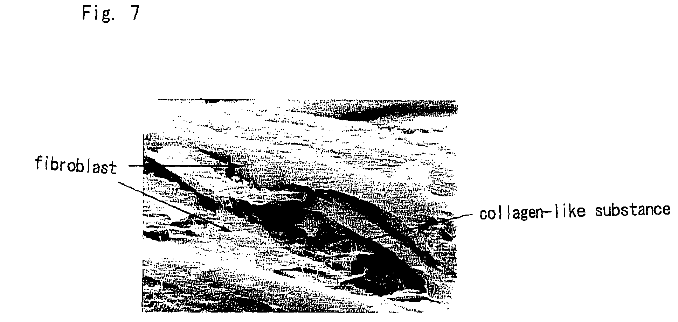

Figure 7 show the image by a scanning electron microscopy obtained when culturing was effected for 28 days using the three dimensional scaffold. Samples were prepared as follows: 0.1 M phosphate buffer (pH7.2) was prepared. To 200 ml of the phosphate buffer, 6.85 g of sucrose was added to prepare 0.1 M phosphate buffer containing 0.1 M sucrose. Likewise, 0.2 M phosphate buffer (pH7.2) containing 0.2 M sucrose was also prepared. In addition, 2 % osmic acid solution was diluted twice with 0.2 M phosphate buffer (pH7.2) containing 0.2 M sucrose to prepare 1 % osmic acid solution.

The scaffolds cultured for 28 days were immersed in 0.1 M phosphate buffer (pH7.2) containing 0.1 M sucrose, and left at 37 °C for 10 minutes. The procedure was repeated three times before the scaffolds were immersed in 2% glutaraldehyde solution (prepared with 0.1 M phosphate buffer), left at 37°C for 1 hour and washed. The scaffolds containing the cells was immersed in 0.1 M phosphate buffer containing 0.1 M sucrose, left at 37°C for 1 hour, and washed. The procedure was repeated three times before the scaffolds were immersed in 1% osmic acid solution at room temperature to be subjected to after-fixation treatment. Further in order to effect conductivity staining, the scaffolds were washed with 0.1 M phosphate buffer before they were immersed in 2 % aqueous tannic acid solution for two hours followed by the immersion in 0.1 M phosphate buffer for two hours. The scaffolds were then immersed in 2 % osmic acid containing 0.34 g/10mL of sucrose for two hours followed by the immersion in 0.1 M phosphate buffer for 2 hours.

The scaffolds were immersed for 10 minutes in aqueous ethanol solutions in an order (20-99.5 % ethanol) in which the concentration of ethanol was increased at 10 % interval. It was finally immersed in absolute ethanol twice to be dehydrated. The scaffolds were then immersed twice in isoamyl acetate for 30 minutes to change solvent from ethanol to isoamyl acetate. Each sample which was subjected to a critical point drying treatment (temperature: 31°C, pressure: 72.8 atm.) using liquidized CO₂ as a shifting solution. Each sample was then subjected to a platinum-palladium vacuum evaporation treatment with an ion-spattering equipment (E-102, Hitachi Ltd.) and then the state of proliferation and differentiation of fibroblasts on the scaffolds was observed. As results, fibroblasts proliferated and differentiated on the surface of the three dimensional scaffolds and much fibrous substance which were considered to be collagen was observed (Figure 7).

## Claims

1. A chitosan/acidic biopolymers hybrid fibers in which the inner part of the fibers comprises chitosan or salts thereof and the surface of the fibers are covered by a complex of chitosan and a biodegradable acidic biopolymers and which retains the form thereof when the fibers are soaked in DMEM medium (Dulbecco's Modified Eagle's Medium) at room temperature for 2 weeks.

2. A chitosan/acidic biopolymers hybrid fibers of Claim 1 in which the acidic biopolymers are selected from the group consisting of hyarulonic acid, alginic acid, chondroitin sufate, dermatan sulfate, heparin, heparan sulfate, keratan sulfate and polyglutamic acid.

3. A method for preparing the fibers of Claim 1 which comprise the steps of:
1) dissolving chitosan in an aqueous acid solution to prepare an aqueous solution of chitosan salt;
2) wet spinning the aqueous solution of chitosan salt using alkaline earth metal salts as coagulants to form fibers;
3) immersing the fibers in a solution of biodegradable acidic biopolymers to react chitosan with acidic biopolymers on the surface of the fibers to form chitosan/acidic biopolymer hybrid fibers;
4) optionally stretching the hybrid fibers; and
5) treating the fibers with bases, di- or more-basic inorganic acids or salts thereof, tri- or more-basic organic acids or salts thereof.

4. A method for preparing the fibers of Claim 1 which comprise the steps of:
1) dissolving chitosan in aqueous acid solutions to prepare aqueous solutions of chitosan salt;
2) wet spinning the aqueous solution of chitosan salt using bases, di- or more-basic inorganic acids or salts thereof, tri- or more organic basic acids or salts thereof as a coagulant to form fibers;
3) immersing the fibers in a solution of biodegrabable acidic biopolymers to react chitosan with the acidic biopolymers on the surface of the fibers to form chitosan/acidic biopolymer hybrid fibers ; and
4) optionally stretching the hybrid fibers.

5. Three dimensional scaffolds for animal cells comprising the fibers of Claim 1.

6. Three dimensional scaffolds of Claim 5 in which the animal cell is chondrocyte.

7. Three dimensional scaffolds of Claim 5 in which the animal cell is fibroblast.

8. Three dimensional scaffolds of Claim 5 in which the animal cells are undifferentiated cells.

9. A method for culturing chondrocytes comprising culturing chondrocytes cells in vitro using the three dimensional scaffolds of Claim 6.

10. A method for culturing chondrocytes of Claim 9 in which a growth factor is added during culturing.

11. A method for culturing of Claim 9 or 10 in which the culturing is effected under a low oxygen condition of 1 to 15 % and / or under a pressure of 0.1 to 20 MPa.

12. A method for culturing fibroblasts comprising culturing fibroblasts in vitro using the three dimensional scaffolds of Claim 7.

13. A method for culturing fibroblasts of Claim 12 in which a growth factor is added during culturing.

14. A method for culturing fibroblasts of Claim 12 or 13 in which culturing is effected with a stretch stimulus of 0.01 to 50 mm/cm being added.

15. A method for culturing animal cells comprising culturing undifferentiated cells in vitro using the three dimensional scaffolds of Claim 8.
